# EUROPÄISCHE PATENTSCHRIFT

(11) **EP 1 002 811 B1**
(45) Veröffentlichungstag und Bekanntmachung des Hinweises auf die Patenterteilung: **13.02.2002**
(21) Anmeldenummer: 99122635.8
(22) Anmeldetag: 13.11.1999
(51) Int. Cl.: C08F 271/02, A61K 7/06

(54) **Wasserlösliche oder wasserdispergierbare Pfropfcopolymerisate auf der Basis eines Polyvinyllactams, deren Herstellung und Verwendung**
Soluble or water-dispersible graft copolymers based on a polyvinyllactam, the preparation and use thereof
Copolymères greffés solubles ou dispersables dans l'eau à base d'un polyvinyllactame, leur préparation et leur utilisation

(30) Priorität: 18.11.1998 DE 19853046
(43) Veröffentlichungstag der Anmeldung: 24.05.2000
(73) Patentinhaber: BASF AKTIENGESELLSCHAFT, 67056 Ludwigshafen (DE)
(72) Erfinder: Nguyen Kim, Son, Dr., 69502 Hemsbach (DE); Sanner, Axel, Dr., 67227 Frankenthal (DE); Hössel, Peter, Dr., 67105 Schifferstadt (DE); Schehlmann, Volker, Dr., 67105 Schifferstadt (DE)

(56) Entgegenhaltungen:
- DE-A- 4 202 193
- US-A- 3 301 808
- US-A- 3 405 084
- US-A- 3 770 683

## Beschreibung

Die Erfindung betrifft Pfropfcopolymerisate, bei denen ein tert.-Butylacrylat und eine polymerisierbare Carbonsäure auf ein Polyvinyllactam aufgepfropft sind, deren Herstellung und deren Verwendung vor allem in Form kosmetischer Zubereitungen, besonders als Haarfestiger.

Als Filmbildner für kosmetische Zubereitungen, insbesondere für die Haarkosmetik, sind bereits eine Vielzahl von Stoffen beschrieben worden. Zum überwiegenden Teil handelt es sich dabei um synthetisch gewonnene Polymere, die in gelöster Form durch Einreiben oder Aufsprühen auf das Haar appliziert werden, wo sie nach Verdunsten des Lösungsmittels einen transparenten farblosen Film hinterlassen.

An derartige Stoffe werden eine Reihe von Anforderungen gestellt: Der Film soll fest auf dem Haar haften und nicht abschuppen oder abstauben. Er soll klar, glanzgebend und nicht feuchtigkeitsempfindlich sein, damit die behandelte Frisur auch bei hoher Luftfeuchtigkeit formbeständig bleibt, nicht klebt und Staub bindet. Andererseits soll sich das Polymere mit einem handelsüblichen Haarwaschmittel rückstandslos auswaschen lassen. Schließlich erfordert die Versprühbarkeit der Filmbildnerlösung eine ausreichende Löslichkeit des Polymeren in den für Haarbehandlungsmittel üblichen Lösungsmitteln und im Falle der Applikation als Spray aus einer Druckpackung die einwandfreie Mischbarkeit der Lösung mit den Treibmitteln.

Während in der Anfangszeit bevorzugt Vinyllactam-Homo- und Copolymere zum Einsatz gelangten, haben später carboxylatgruppenhaltige Polymere zunehmend an Bedeutung gewonnen. Das gewünschte Eigenschaftsprofil wie starke Festigung bei hoher Luftfeuchtigkeit, Elastizität, Auswaschbarkeit vom Haar und Verträglichkeit mit den übrigen Formulierungskomponenten werden durch Copolymerisation einer Kombination von hydrophoben, elastifizierenden und Carboxylgruppen enthaltenden Monomeren erzielt.

Während die obengenannten Anforderungen heute von verschiedenen Polymertypen erreicht werden, wird immer häufiger der Griff der mit diesen Polymeren gefestigten Frisuren als unangenehm stumpf und "unnatürlich" empfunden. Versuche, durch Zusätze zu den Formulierungen zu einer Verbesserung zu gelangen, führten bisher nicht zu voll befriedigenden Ergebnissen: Die Zugabe üblicher Weichmacher verbessert zwar den Griff, reduziert aber gleichzeitig in vielen Fällen die Festigungswirkung. Die häufig eingesetzten Polysiloxane sind mit den polaren Polymeren nicht verträglich und verlangen oft weitere Zusätze, um überhaupt formuliert werden zu können. Entmischungen können sowohl während der Lagerung der Formulierung als auch während des Gebrauchs zu Problemen führen.

Dies gilt z.B. für die in US 3,405,084 beschriebenen Terpolymere, die 25 bis 75 % Vinylpyrrolidon, 20 bis 70 % Acrylat und 3 bis 25 % Acrylsäure enthalten.

Verbesserte Terpolymerisate gemäß EP-A 0 257 444 enthalten 20 bis 50 % Vinylpyrrolidon, 40 bis 70 % tert.-Butylacrylat und 2 bis 15 % Acrylsäure oder Methacrylsäure.

Ferner sind in der US-A 3,594,344 Pfropfcopolymere für Haarspray-Formulierungen beschrieben, die durch Pfropfung einer Monomermischung aus Alkylacrylaten und mindestens 1 Gew.-% Glycidylmethacrylat auf polymeres N-Vinyllactam hergestellt wurden. Nachteilig. bei diesen Polymerisaten ist aber nicht nur die Toxizität des Glycidylmethacrylats, sondern auch die hohe Vernetzungstendenz dieses bifunktionalen Monomeren. Dies kann z.B. zur Lagerinstabilität der Dispersion führen. Darüber hinaus können die Epoxidgruppen des Polymeren eine dauerhafte, chemische Bindung mit der Eiweißsubstanz des Haares und des Haarbodens eingehen. Derartige Polymere sind nicht auswaschbar und werden als physiologisch bedenklich abgelehnt.

In der US-A 3,770,683 werden Emulsionspfropfcopolymere für Haarspray-Formulierungen beschrieben, die durch Pfropfung einer Monomerenmischung aus (Meth)acrylsäureestern und mindestens 0,5 Gew.-% einer ethylenisch ungesättigten Monocarbonsäure auf polymeres N-Vinyllactam hergestellt wurden.

Es hat nun nicht an Versuchen gefehlt, die Eigenschaften dieser Polymere dahingehend zu verbessern, daß sie weniger feuchtigkeitsempfindlich werden und vor allem in Gegenden mit feuchtheißem Klima anwendbar sind.

Demgemäß wurde schon gemäß DE-A 42 02 193 vorgeschlagen, Emulsionspfropfcopolymerisate aus Polyvinyllactamen mit aufgepfropften Acrylsäureestern für die Haarkosmetik zu verwenden. Dabei wurde festgestellt, daß es nicht vorteilhaft ist, Monomere mit Carboxylgruppen einzupolymerisieren, wie dies in US 3,770,683 beschrieben ist, da die für die Wasserlöslichkeit erforderliche Neutralisation bzw. Teilneutralisation nur einen engen Spielraum läßt, so daß man leicht Filme erhält, die entweder schlecht auswaschbar oder weich und feuchtigkeitsempfindlich sind.

Aufgabe der vorliegenden Erfindung war nun, weiter verbesserte Pfropfpolymerisate vorzuschlagen, die weder feuchtigkeitsempfindlich sind noch wegen ihrer Härte beim Auskämmen auf dem Haar einen schuppenförmigen Rest zurücklassen und auch in heißfeuchtem Klima anwendbar sind.

Diese Aufgabe wurde erfindungsgemäß mit speziellen Pfropfpolymerisation gelöst, wobei überraschenderweise der Gehalt an Carboxylgruppen enthaltenden Comonomeren nicht zu den in DE-A 42 02 193 geschilderten Nachteilen führt.

Gegenstand der Erfindung sind demgemäß wasserlösliche oder wasserdispergierbare Pfropfcopolymerisate mit einem K-Wert von 30 bis 70, erhältlich durch radikalische Polymerisation von Monomeren
(A) bestehend im wesentlichen aus
   (a) 50 bis 85 Gew.-% von Monomeren der Formel I 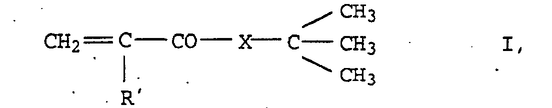 in der R' für Wasserstoff oder C₁- bis C₆-Alkylreste und X für die Reste 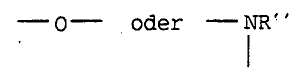 steht, wobei R'' Wasserstoff oder einen C₁- bis C₆-Alkylrest bedeutet, und
   (b) 15 bis 30 Gew.-% eines mindestens eine Carboxylgruppe aufweisenden Vinylmonomers, sowie gegebenenfalls
   (c) 0 bis 25 Gew.-% eines radikalisch polymerisierbaren Vinylmonomers der Formel II 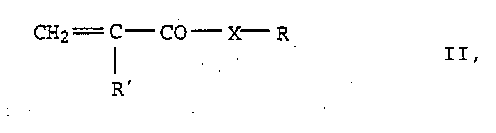 in der R einen C₆-C₃₀-Alkylrest, vorzugsweise C₆-C₂₂-Alkylrest, bedeutet und R' und X die obengenannten Bedeutungen haben, mit
(B) einem Präpolymer mit einem K-Wert von 30 bis 50, enthaltend einpolymerisiert mindestens 30 Gew.-% von Monomeren der Formel III 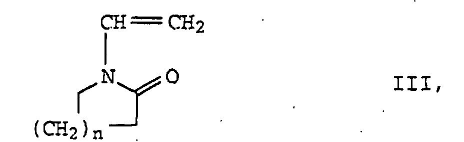 in der n die Zahl 1 bis 3 bedeutet, wobei das
   Gewichtsverhältnis A:B 100:5 bis 100:200 beträgt und zumindest Teilneutralisation des Pfropfcopolymers.

Bei der Komponente A(a) handelt es sich bevorzugt um α,β-ethylenisch ungesättigte Verbindungen der allgemeinen Formel I, worin
- R': für Wasserstoff, Methyl oder Ethyl steht und
- X: für O oder NR'' steht, wobei R" für Wasserstoff, Methyl, Ethyl, n-Propyl, Isopropyl, n-Butyl, sec.-Butyl, tert.-Butyl oder Cyclohexyl steht,

Dabei können auch Mischungen von Verbindungen der Komponente A(a) eingesetzt werden.

Bevorzugt handelt es sich bei der Komponente A(a) um tert.-Butylacrylat, tert.-Butylmethacrylat, tert.-Butylethacrylat, N-tert.-Butylacrylamid, N-tert.-Butylmethacrylamid, N-tert.-Butylethacrylamid und Mischungen davon.

Bevorzugt kommen tert.-Butylmethacrylat und insbesondere tert.-Butylacrylat in Betracht.

Monomere (A)(b) sind beliebige, vor allem niedermolekulare, eine oder mehrere Carboxylgruppen tragende Vinylgruppen enthaltende Verbindungen. Im einzelnen seien z.B. Acrylsäure, Methacrylsäure, Fumarsäure, Maleinsäure, Itaconsäure, Crotonsäure und Mischungen davon genannt. Bevorzugt werden Acrylsäure, Methacrylsäure und Mischungen davon eingesetzt.

Als Monomere (A)(c) der Formel II kommen vorzugsweise solche in Betracht, bei denen R' Wasserstoff, Methyl oder Ethyl, X=O oder NH und R die Reste n-Octyl, Ethylhexyl, 1,1,3,3-Tetramethylbutyl, Undecyl, Lauryl, Tridecyl, Myristyl, Pentadecyl, Palmityl, Margarinyl, Stearyl, Palmitoleinyl, Oleyl oder Linolyl bedeutet.

Insbesondere ist die Komponente (A) (c) ausgewählt unter n-Octyl (meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)-acrylat, Pentadecyl(meth)acrylat, Palmityl(meth)acrylat, Heptadecyl(meth)acrylat, Nonadecyl (meth)acrylat, Arrachinyl(meth)-acrylat, Behenyl(meth)acrylat, Lignocerenyl(meth)acrylat, Cerotinyl(meth)acrylat, Melissinyl(meth)acrylat, Palmitoleinyl-(meth)acrylat, Oleyl(meth)acrylat, Linolyl(meth)acrylat, Linolenyl(meth)acrylät, Stearyl(meth)acrylat, Lauryl(meth)-acrylat, n-Octyl(meth)acrylamid, 1,1,3,3-Tetramethylbutyl(meth)-acrylamid, Ethylhexyl(meth)acrylamid, n-Nonyl(meth)acrylamid, n-Decyl(meth)acrylamid, n-Undecyl(meth)acrylamid, Tridecyl(meth)-acrylamid, Myristyl(meth)acrylamid, Pentadecyl(meth)acrylamid, Palmityl(meth)acrylamid, Heptadecyl(meth)acrylamid, Nonadecyl-(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl-(meth)acrylamid, Linolyl (meth)acrylamid, Linolenyl(meth)acrylamid, Stearyl(meth)acrylamid, Lauryl(meth)acrylamid und Mischungen davon.

Insbesondere sind Stearyl(meth)acrylamid, Lauryl(meth)acrylat, Stearyl(meth)acrylat und vorzugsweise Stearylacrylat zu nennen.

Das Ausgangspräpolymer (B) enthält mindestens 30 Gew.-% eines Vinyllactams, vorzugsweise Vinylpyrrolidon oder Vinylcaprolactam. Beispiele sind reines Polyvinylpyrrolidon oder Polyvinylcaprolactam oder Mischungen davon oder Copolymere, die Vinylpyrrolidon und/oder Vinylcaprolactam in beliebigem Gewichtsverhältnis einpolymerisiert enthalten.

Entsprechende Copolymere enthalten als weitere Comonomere bevorzugt einen Ester oder auch Amide der (Meth)acrylsäure (M1) und/ oder ein tert.-Amin enthaltendes Monomer (M2), wobei der Gehalt an M1 bis 50 Gew.-% und von M2 bis 20 Gew.-% betragen kann.

Das Präpolymer (B) ist entweder ein Homopolymerisat des Vinylcaprolactams oder Vinylpyrrolidons, ein Copolymerisat aus Vinylcaprolactam und Vinylpyrrolidon oder ein Copolymerisat aus Vinylcaprolactam und/oder Vinylpyrrolidon mit einem C₁-C₃₀-Alkylester oder Alkylamid der Acryl- oder Methacrylsäure (M1) und/oder einem tert.-Amin haltigen Monomer (M2) der Formel V 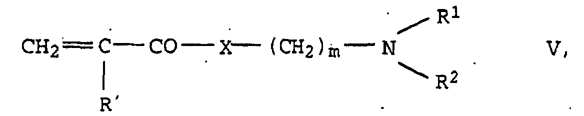 in der R' Wasserstoff oder einen C₁- bis C₆-Alkylrest, bevorzugt Wasserstoff oder Methyl, R¹ und R² unabhängig voneinander einen Alkylrest mit 1 bis 6 C-Atomen, vorzugsweise Methyl, X Sauerstoff oder eine Iminogruppe und m die Zahlen 2 bis 8, vorzugsweise 2 oder 3, bedeuten.

Bevorzugt zu verwendende Monomere der Formel V sind Dimethylaminoethylacrylat, Dimethylaminoethylmethacrylat Dimethylaminopropylacrylat, Dimethylaminopropylmethacrylat Dimethylaminoethylacrylamid, Dimethylaminoethylmethacrylamid Dimethylaminopropylacrylamid, Dimethylaminopropylmethacrylamid.

Als Copolymerisat von Vinylpyrrolidon mit einem C₁-C₃₀-Alkylacrylat sind Copolymere mit tert.-Butylacrylat bevorzugt.

Das Gewichtsverhältnis der aufzupfropfenden Monomere (A) zum Präpolymer (B) beträgt 100:5 bis 100:200, vorzugsweise 100:10 bis 100:100 und insbesondere 100:20 bis 100:70.

Die erfindungsgemäßen Pfropfcopolymerisate werden in an sich bekannter Weise durch radikalisch initiierte Suspensions-, Emulsions- und bevorzugt Lösungspolymerisation durch Aufpfropfen der Monomere (A) auf das Präpolymer (B) hergestellt. Dabei kann auch ein Teil der aufgepfropften Monomerkomponente im resultierenden Pfropfcopolymerisat als isolierte Polymerketten vorliegen, ohne daß Pfropfung stattgefunden hat.

Als Initiatoren für die radikalisch ablaufende Polymerisationsreaktion werden die üblichen Peroxo- oder Azoverbindungen, beipielsweise Dibenzoylperoxid, tert.-Butylperpivalat, tert.-Butylper-2-ethylhexanoat, Di-tert.-butylperoxid, tert.-Butylhydroperoxid, 2,5-Dimethyl-2,5-di(tert.-butylperoxy)hexan, Alkalimetall oder Ammoniumpersulfate, Azo-bis-butyronitril, Wasserstoffperoxid oder Redoxinitiatoren zweckmäßigerweise in Mengen von 0,1 bis 2 Gew.-%, bezogen auf das Gewicht der Monomeren, eingesetzt. Man wählt die Menge an Monomeren und Lösungs- bzw. Dispergiermittel zweckmäßigerweise so, daß man 30- bis 80 gew.-%ige Lösungen der Copolymerisate erhält. Zur Senkung des Restmonomerengehaltes kann eine Nachpolymerisation nach allgemein bekannten Verfahren erfolgen.

Die Copolymerisate sollen K-Werte von 25 bis 60, vorzugsweise 30 bis 50 aufweisen. Der jeweils gewünschte K-Wert läßt sich in an sich bekannter Weise durch Wahl der Polymerisationsbedingungen, beispielsweise der Polymerisationstemperatur und der Initiatorkonzentration, einstellen. Gegebenenfalls kann der Einsatz von Reglern, insbesondere von Schwefelverbindungen wie Mercaptoethanol, 2-Ethylhexylthioglykolat, Thioglykolsäure oder Dodecylmercaptan zur Reduzierung des K-Wertes angebracht sein. Die K-Werte werden nach Fikentscher, Cellulosechemie, Bd. 13, S. 58 bis 64 (1932) bei 25°C in 1 gew.-%iger ethanolischer Lösung gemessen und stellen ein Maß für das Molgewicht dar.

Derartige Copolymerisate haben üblicherweise Glastemperaturen zwischen 50 und 130°C, insbesondere zwischen 60 und 100°C.

Als Emulgatoren und Schutzkolloide, welche hauptsächlich als Dispergiermittel dienen, eignen sich insbesondere anionische Tenside wie die Alkalimetallsalze von Fettsäuren (Seifen) oder die Alkalimetallsalze von Alkylsulfaten, z.B. Natriumlaurylsulfat, aber auch nichtionische und kationische Emulgatoren bzw. Schutzkolloide, z.B. Polyethylenoxide, Polyoxyethylen-Polyoxypropylen-Blockcopolymere und quarternäre Ammoniumsalze wie Cetyltrimethylammoniumbromid, lassen sich einsetzen.

Weiterhin können dem Reaktionsgemisch weitere übliche Hilfsstoffe zugegeben werden, wie z.B. Puffersubstanzen, Komplexbildner oder Elektrolytzusätze zur weiteren Herabsetzung der Viskosität.

Die erhaltenen wäßrigen Dispersionen werden je nach dem Gehalt an Monomeren (A)(b) teilneutralisiert oder vollständig neutralisiert, um die Pfropfcopolymerisate wasserlöslich oder zumindest wasserdispergierbar zu machen. Den Umfang der Neutralisation läßt sich im Einzelfall leicht am Ergebnis der Wasserlöslichkeit bzw. Wasserdispergierbarkeit feststellen. Zur Neutralisation werden in der Regel Alkali- oder Erdalkalibasen oder Amine verwendet.

Die Neutralisation erfolgt bevorzugt mit
- einem Mono-, Di- oder Trialkanolamin mit 2 bis 5 C-Atomen im Alkanolrest, der gegebenenfalls in veretherter Form vorliegt, beispielsweise Mono-, Di- und Triethanolamin, Mono-, Di-und Tri-n-propanolamin, Mono-, Di- und Triisopropanolamin, 2-Amino-2-methylpropanol und Di (2-methoxyethyl)amin,
- einem Alkandiolamin mit 2 bis 5 C-Atomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 C-Atomen, beispielsweise N,N-Diethylpropylamin.

Besonders gute Ergebnisse erhielt man mit 2-Amino-2-methylpropanol, Triisopropanolamin und 2-Amino-2-ethylpropan-1,3-diol.

Als Alkalimetallhydroxide eignen sich zur Neutralisation vor allem Natrium- und Kaliumhydroxid.

Die erfindungsgemäßen Pfropfcopolymerisate sind ausgezeichnete Filmbildner in haarkosmetischen Zubereitungen, vor allem in Haarfestigungsmitteln wie Haarsprays. wäßrigen Haarfestigerlösungen, Haargelen oder Haarschäumen. Sie lassen sich ohne weitere Zusätze an Basen und Hydrophobierungsmitteln auf das Haar applizieren, verfestigen dieses elastisch, verleihen ihm Glanz, haften gut, vermitteln weder Hygroskopizität noch Feuchtigkeitsempfindlichkeit, zeigen eine verminderte Klebrigkeit bei hoher Luftfeuchtigkeit und lassen sich mit handelsüblichen Haarwaschmitteln einwandfrei aus dem Haar entfernen.

Entsprechende Haarkosmetikpräparate enthalten in der Regel 0,1 bis 20 Gew.-%, vorzugsweise 0,5 bis 10 Gew.-%, und insbesondere 2 bis 10 Gew.-%, der teilweise oder vollständig neutralisierten neuen Pfropfcopolymerisate neben üblichen Lösungsmitteln, wie Wasser und/oder Alkoholen sowie gegebenenfalls Treibmittel, z.B.
- 1 bis 99,9 Gew.-%, vorzugsweise 5 bis 50 Gew.-%, insbesondere 10 bis 40 Gew.-%, Wasser,
- 0 bis 95 Gew.-%, vorzugsweise 20 bis 60 Gew.-%, insbesondere 25 bis 50 Gew.-%, eines üblichen organischen Lösungsmittels, wie vor allem Ethanol, Isopropanol und Dimethoxymethan und daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische,
- 0 bis 90 Gew.-%, vorzugsweise 30 bis 80 Gew.-%, insbesondere 45 bis 70 Gew.-%, eines üblichen Treibmittels wie Propan, n-Butan, Isobutan, 2,2-Dimethylbutan, n-Pentan, Isopentan, Dimethylether, Difluorethan, Fluortrichlormethan, Dichlordifluormethan oder Dichlortetrafluorethan oder deren Gemische.

Die Haarfestigerzusammensetzungen können zusätzlich 0 bis 10 Gew.-% eines handelsüblichen Haarpolymers, 0 bis 0,5 Gew.-% einer wasserlöslichen oder dispergierbaren Silikonverbindung sowie 0 bis 0,2 Gew.-% üblicher Additive enthalten.

Die erfindungsgemäßen Pfropfpolymerisate können auch in Kombination mit anderen Haarpolymeren z. B. 0 bis 10 Gew.-% eines handelsüblichen Haarpolymers zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen;
- amphotere oder zwitterionische Polymere, sowie die unter den Bezeichnungen Amphomer® (Delft National) erhältlichen Octylacrylamid/Methylmethacrylat/ter.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methylacrylsäure-Copolymerisate und deren Alkali- und Ammoniumsalze sind bevorzugte zwitterionische Polymere. Weiterhin geeignete zwitterionische Polymere sind Methacrylethylbetain/Methäcrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder anionische Polyurethane wie sie z.B. aus DE-A 4 225 045 und EP-A 619 111 oder silikonhaltige Polyurethane wie sie aus EP-A 636 361 bekannt sind;
- kationische (quaternisierte) Polymere, z.B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von PolyVinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische siloxanhaltige, wasserlösliche oder -dispergierbare Polymere, z. B. Polyethersiloxane, wie Tegopren® (Fa. Goldschmidt) oder Belsil® (Fa. Wacker).

Weiter enthalten die erfindungsgemäßen Haarbehandlungsmittel im allgemeinen übliche kosmetische Hilfsstoffe, beispielsweise Weichmacher, wie Glycerin und Glykol; Emollienzien; Parfüms; UV-Adsorber; Farbstoffe; antistatische Mittel; Mittel zur Verbesserung der Kämmbarkeit; Konservierungsmittel; und Entschäumer.

Als Treibmittel (Treibgase) kommen von den genannten Verbindungen vor allem die Kohlenwasserstoffe, insbesondere Propan, n-Butan, n-Pentan und Gemische hieraus, sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Demgemäß enthält ein erfindungsgemäßes Haarfestigerpräparat z. B.
- 0,5 bis 20 Gew.-%: eines erfindungsgemäßen Pfropfcopolymerisats,
- 0 bis 10 Gew.-%: eines handelsüblichen Haarpolymers
- 50 bis 99,5 Gew.-%: eines Alkohols und/oder Wasser als Lösungsmittel,
- 0 bis 50 Gew.-%: eines Treibmittels in Form von Propan/Butan und/oder Dimethylether.
- 0 bis 0,5 Gew.-%: einer wasserlöslichen oder dispergierbaren Silikonverbindung und
- 0 bis 0,2 Gew.-%: üblicher Additive.

Die erfindungsgemäßen Haarfestigungsmittel eignen sich auch besonders für Pumpsprayzubereitungen ohne Zusatz von Treibmitteln oder auch für Aerosolsprays mit üblichen Druckgasen wie Stickstoff, Druckluft oder Kohlendioxid als Treibmittel.

Außerdem können diese Sprayzubereitungen noch geringe Mengen an Parfümölen, beispielsweise 0,1 bis 0,5 Gew.-%, enthalten.

Eine wasserhaltige Standard-Sprayformulierung weist beispielsweise die folgende Zusammensetzung auf:
- 2 bis 10 Gew.-%: des zu 100 % mit 2-Amino-2-methylpropanol neutralisierten Pfropfcopolymerisates,
- 10 bis 76 Gew.-%: Ethanol,
- 2 bis 40 Gew.-: Wasser,
- 10 bis 40 Gew.-%: Dimethylether.

Die in erfindungsgemäßen Haarfestigungsmitteln enthaltenen Pfropfcopolymerisate zeichnen sich durch ihre hohe Verträglichkeit mit den unpolaren Treibmitteln in Sprayzubereitungen, insbesondere mit Kohlenwasserstoffen wie Propan oder n-Butan, oder ihrem Gemisch aus. Sie weisen eine gute haarfestigende Wirkung auf, ersichtlich an den hohen Werten für die Curl-Retention, die hierbei meist über 80 % liegen. Außerdem zeichnen sich die erfindungsgemäßen Haarfestigungsmittel dadurch aus, daß sie das Haar praktisch nicht verkleben.

Vor allem aber weisen die erfindungsgemäßen Haarfestigungsmittel bei den anwendungstechnischen Eigenschaften, die eingangs bei den Mitteln des Standes der Technik als verbesserungsbedürftig bezeichnet wurden, hervorragende Ergebnisse auf. Sie sind in Alkoholen wie Ethanol oder Isopropanol und in Gemischen dieser Alkohole mit Wasser klar löslich. Die Klarheit der Lösungen bleibt auch erhalten, wenn die Lösungen in Standard-Sprayformulierungen zusammen mit Treibmitteln wie Dimethylether eingesetzt werden. Die erfindungsgemäßen Haarfestigungsmittel sind einwandfrei aus dem Haar auswaschbar. Mit ihnen behandeltes Haar weist eine erhöhte Geschmeidigkeit und einen angenehmen natürlichen Griff auf. Die Festigungswirkung ist gleichzeitig dabei hoch, so daß prinzipiell eine Senkung der benötigten Menge an Filmbildner in der Haarsprayformulierung möglich ist.

Ferner eignen sich die erfindungsgemäßen Pfropfcopolymerisate als Beschichtungs- oder Bindemittel in pharmazeutischen Zubereitungen.

### Beispiele

### Beispiel 1

### a) Herstellung des Präpolymers

- Vorlage:: 65,0 g Ethanol 32,0 g der Gesamtmenge Zulauf 1 10,0 g der Gesamtmenge Zulauf 2
- Zulauf 1:: 150 g Vinylcaprolactam (Vcap)
- Zulauf 2:: 80,0 g Ethanol 0,4 g t-Butylperpivalat
- Zulauf 3:: 80,0 g Ethanol 1,6 g t-Butylperpivalat

Man erhitzte unter Rühren die vorgelegte Mischung unter Stickstoff in einer Rührapparatur mit 3 Zulaufeinheiten auf 80°C. Dann wurden die Zuläufe gestartet und der Zulauf 1 innerhalb von 3 Stunden und Zulauf 2 innerhalb von 4 Stunden zudosiert. Nachdem 5 Stunden bei 80°C nachpolymerisiert worden war, wurde Zulauf 3 gestartet und innerhalb von einer Stunde zudosiert. Nach erneutem 5-stündigem Nachpolymerisieren wurde eine 40%ige ethanolische Polyvinylcaprolactam-Lösung erhalten.

Analog wurden die Präpolymere B2 bis B4 der Tabelle 1 hergestellt:

**Tabelle 1**

| Präpolymer | VCap Gew.-% | VP Gew.-% | DMAPMA Gew.-% | TBA Gew.-% |
|---|---|---|---|---|
| B2 | 50 | 50 | - | - |
| B3 | 35 | 35 | 30 | - |
| B4 | - | 65 | - | 35 |
| VP : Vinylpyrrolidon | | | | |
| Vcap: Vinylcaprolactam | | | | |
| DMAPMA : Dimethylamino-propylmethacrylamid | | | | |
| TBA : tert.-Butylacrylat | | | | |

### b) Herstellung der Pfropfpolymere

- Vorlage:: 32,0 g der Gesamtmenge Zulauf 1 10,0 g der Gesamtmenge Zulauf 2 375,0 g (40%ig in Ethanol) Lösung B1.
- Zulauf 1:: 60,0 g MAS 240,0 g TBA
- Zulauf 2:: 100,0 g Ethanol 0,4 g t-Butylperpivalat
- Zulauf 3:: 150,0 g Ethanol 1,6 g t-Butylperpivalat
- Zulauf 4:: 200,0 g Ethanol

Man erhitzte unter Rühren die vorgelegte Mischung unter Stickstoff in einer Rührapparatur mit 4 Zulaufeinheiten auf 80°C. Dann wurden die Zuläufe 1 und 2 gestartet und Zulauf 1 innerhalb von 3 Stunden und Zulauf 2 innerhalb von 4 Stunden zudosiert. Nachdem 5 Stunden bei 80°C nachpolymerisiert worden war, wurde Zulauf 3 gestartet und innerhalb einer Stunde bei 80°C zudosiert und die Mischung 5 Stunden bei 80°C nachpolymerisiert. Beim Abkühlen wurde mit Zulauf 4 verdünnt und man erhielt eine ethanolische Polymerlösung, die unmittelbar zur Herstellung von Haarkosmetika verwendet werden kann.

Analog wurden die Pfropfcopolymere der Beispiele 2 bis 8 gemäß der folgenden Tabelle 2 erhalten.

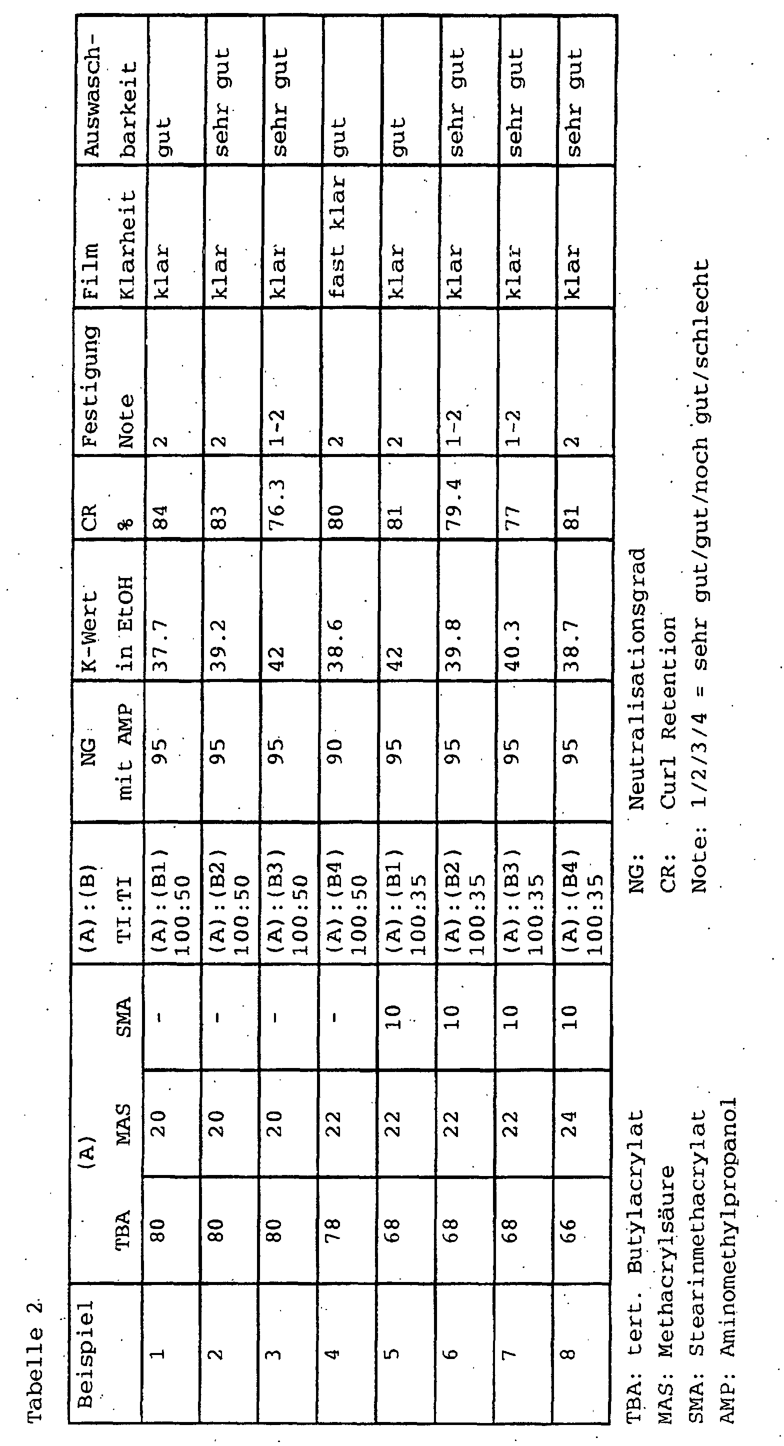

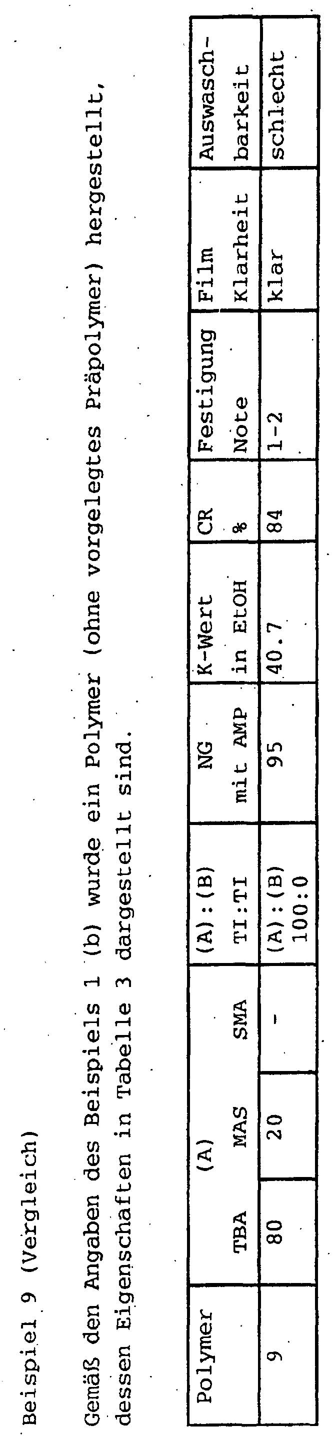

Zum Vergleich wurde dieser Polymer 9, reines Präpolymer B1 und die Mischung aus beiden in Tabelle 4 untersucht und bewertet:

**Tabelle 4**

| | Polymer Nr. 9 | Polymer Nr. B1 | Film |
|---|---|---|---|
| 1 | 100 TL | 0 TL | klar; glatt |
| 2 | 0 | 100 | klar; glatt |
| 3 Mischung statt Pfropfpolymerisat; zum Vergleich | 100 TL | 50 TL | trüb; matt => Unverträglichkeit |

### Beispiele 10 bis 13 (Anwendung als Haarfestiger)

| Beispiel 10 | |
|---|---|
| Aerosol-Haarspray | [ % ] |
| Polymer Nr. 1-8 | 3.00 |
| Dimethylether | 50.00 |
| Ethanol | 47.00 |
| Weitere Zusätze Silkon, Parfüm, Entschäumer etc. | |

| Beispiel 11 | |
|---|---|
| Aerosol-Haarspray | [ % ] |
| Polymer Nr. 1-8 | 5.00 |
| Pro/Bu-Gas | 50.00 |
| Ethanol | 45.00 |
| Weitere Zusätze Silkon, Parfüm, Entschäumer etc. | |

| Beispiel 12 | |
|---|---|
| VOC 80 Aerosol-Haarspray | [ % ] |
| Polymer Nr. 1-8 | 5.00 |
| Wasser | 15.00 |
| Dimethylether | 40.00 |
| Ethanol | 40.00 |
| Weitere Zusätze Silkon, Parfüm, Entschäumer etc. | |

| Beispiel 13 | |
|---|---|
| VOC 55 Handpumpe-Spray | [ % ] |
| Polymer Nr. 1 - 8 | 5.00 |
| Wasser | 40.00 |
| Ethanol | 55.00 |
| Weitere Zusätze Silkon, Parfüm, Entschäumer etc. | |
| (VOC = Volatile Organic compounds in Prozent) | |

## Patentansprüche

1. Wasserlösliche oder wasserdispergierbare Pfropfcopolymerisate mit einem K-Wert von 30 bis 70, erhältlich durch radikalische Polymerisation von Monomeren
(A) bestehend im wesentlichen aus
(a) 50 bis 85 Gew.-% von Monomeren der Formel I 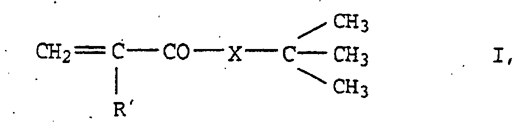 in der R' für Wasserstoff oder einen C₁- bis C₆-Alkylrest und X für die Reste 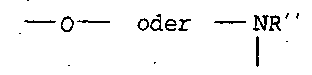 steht, wobei R'' Wasserstoff oder einen C₁- bis C₆-Alkylrest bedeutet, und
(b) 15 bis 30 Gew.-% eines mindestens eine Carboxylgruppe aufweisenden Vinylmonomers, sowie gegebenenfalls
(c) 0 bis 25 Gew.-% eines radikalisch polymerisierbaren. Vinylmonomers der Formel II 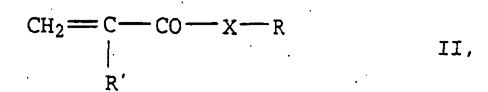 in der R einen C₆- bis C₃₀-Alkylrest bedeutet und R' und X die obengenannten Bedeutungen haben, mit
(B) einem Präpolymer mit einem K-Wert von 30 bis 50, enthaltend einpolymerisiert mindestens 30 Gew.-% von Monomeren der Formel III 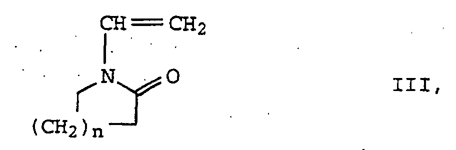 in der n die Zahl 1 bis 3 bedeutet, wobei das Gewichtsverhältnis A:B 100:5 bis 100:200 beträgt und zumindest Teilneutralisation des Pfropfcopolymers.

2. Wasserlösliche oder wasserdispergierbare Pfropfcopolymerisate nach Anspruch 1, **dadurch gekennzeichnet, daß** die Monomeren
(A) zu
(a) 65 bis 85 Gew.-% aus tert.-Butylacrylat, zu
(b) 15 bis 25 Gew.-% aus Methacrylsäure und zu
(c) 0 bis 10 Gew.-% aus Stearylmethacrylat bestehen und auf ein Präpolymer
(B) der Formel IV
―(Vcap)―(VP)―(M1)―(M2)― IV
aufpolymerisiert sind, wobei Vcap Vinylcaprolactam, VP Vinylpyrrolidon, M1 einen Ester der Methacrylsäure oder Acrylsäure mit einem C₁- bis C₃₀-Alkohol oder ein C₁-C₃₀-Alkylamid der Methacrylsäure oder Acrylsäure und M2 ein tert.-Amin haltiges Monomer bedeutet, wobei der Anteil
von Vcap 0 bis 100 Gew.-%,
von VP 0 bis 100 Gew.-%,
von M1 0 bis 50 Gew.-%, und
von M2 0 bis 20 Gew.-%
beträgt, mit der Maßgabe, daß die Summe aus Vcap und VP mindestens 30 Gew.-% und das Massenverhältnis (A) zu (B) 100:20 bis 100:70 beträgt.

3. Wasserlösliche oder wasserdispergierbare Pfropfcopolymerisate nach Anspruch 1, **dadurch gekennzeichnet, daß** das Präpolymer (B) Polyvinylcaprolactam ist.

4. Wasserlösliche oder wasserdispergierbare Pfropfcopolymere nach Anspruch 1, **dadurch gekennzeichnet, daß** das Präpolymer (B) Polyvinylpyrrolidon ist.

5. Wasserlösliche oder wasserdispergierbare Pfropfcopolymerisate nach Anspruch 1, **dadurch gekennzeichnet, daß** das Präpolymer (B) ein Copolymerisat aus Vinylpyrrolidon- und Vinylcaprolactam ist.

6. Wasserlösliche oder wasserdispergierbare Pfropfcopolymerisate nach Anspruch 1, **dadurch gekennzeichnet, daß** das Präpolymer (B) ein Copolymerisat aus Vinylpyrrolidon und/oder Vinylcaprolactam und einem eine tertiäre Amingruppe enthaltenden Monomer ist.

7. Wasserlösliche oder wasserdispergierbare Pfropfcopolymerisate nach Anspruch 6, **dadurch gekennzeichnet, daß** das eine tertiäre Amingruppe enthaltende Monomer ein Monomer der Formel V 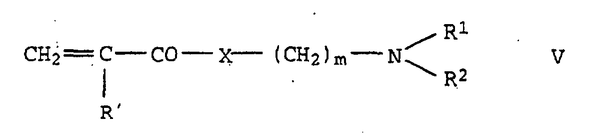 ist, in der R' Wasserstoff oder einen C₁- bis C₆-Alkyrest, X Sauerstoff oder eine Iminogruppe, m die Zahlen 2 bis 8 und R¹ und R² unabhängig voneinander einen Alkylrest mit 1 bis 6 C-Atomen bedeuten.

8. Verfahren zur Herstellung von Pfropfcopolymerisaten gemäß Anspruch 1, **dadurch gekennzeichnet, daß** man die Monomere
(A), bestehend aus
(a) 50 bis 85 Gew.-% von Monomeren der Formel I 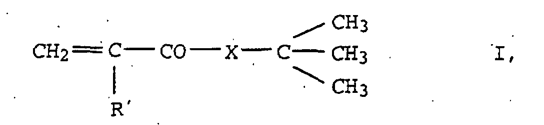 in der R' für Wasserstoff oder für einen C₁- bis C₆-Alkylrest und X für die Reste 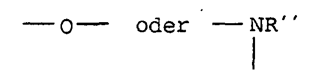 steht, wobei R'' Wasserstoff oder einen C₁- bis C₆-Alkylrest bedeutet, und
(b) 15 bis 30 Gew.-% eines mindestens eine Carboxylgruppe aufweisenden Vinylmonomers, sowie gegebenenfalls
(c) 0 bis 25 Gew.-% eines radikalisch polymerisierbarer Vinylmonomers der Formel II 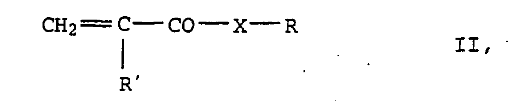 in der R einen C₆- bis C₃₀-Alkylrest bedeutet und R' und X die obengenannten Bedeutungen haben, mit
(B) einem Präpolymer mit einem K-Wert von 30 bis 50, enthaltend einpolymerisiert mindestens 30 Gew.-% von Monomeren der Formel III 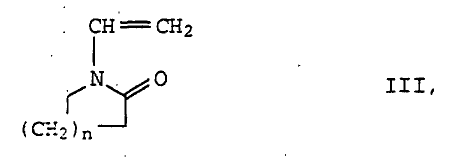 in der n die Zahl 1 bis 3 bedeutet, wobei das
Gewichtsverhältnis A:B 100:5 bis 100:200 beträgt, radikalisch polymerisiert und zumindest einen Teil der Carboxylgruppen des Pfropfcopolymers neutralisiert.

9. Verwendung der wasserlöslichen Pfropfcopolymere gemäß einem der Ansprüche 1 bis 7 als Bestandteil von kosmetischen Zubereitungen.

10. Haarkosmetikpräparat, enthaltend 0,5 bis 20 Gew.-% eines Pfropfcopolymerisats gemäß Ansprüchen 1 bis 7.

11. Verwendung der wasserlöslichen Pfropfcopolymere gemäß einem der Ansprüche 1 und 7 als Beschichtungs- oder Bindemittel in pharmazeutischen Zubereitungen.

12. Haarfestigerpräparat, enthaltend
0,5 bis 20 Gew.-% eines Pfropfcopolymerisats gemäß einem der Ansprüche 1 bis 7,
0 bis 10 Gew.-% eines handelsüblichen Haarpolymers,
50 bis 99,5 Gew.-% eines Alkohols und/oder Wasser als Lösungsmittel,
0 bis 50 Gew.-% eines Treibmittels in Form von Propan/ Butan und/oder Dimethylether,
0 bis 0,5 Gew.-% einer wasserlöslichen oder dispergierbaren Silikonverbindung und
0 bis 0,2 Gew.-% üblicher Additive.

## Claims

1. A water-soluble or water-dispersible graft copolymer with a K value of from 30 to 70, obtainable by free-radical polymerization of monomers
(A) consisting essentially of
(a) from 50 to 85% by weight of monomers of the formula I 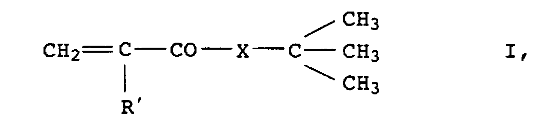 in which R' is hydrogen or a C₁- to C₆-alkyl radical, and X is the radical 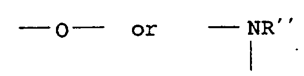 where R'' is hydrogen or a C₁- to C₆-alkyl radical, and
(b) from 15 to 30% by weight of a vinyl monomer which has at least one carboxyl group, and, where appropriate,
(c) from 0 to 25% by weight of a free-radically polymerizable vinyl monomer of the formula II 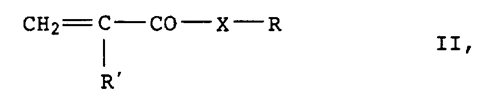 in which R is a C₆- to C₃₀-alkyl radical, and R' and X are as defined above, with
(B) a prepolymer with a K value of from 30 to 50, comprising, in copolymerized form, at least 30% by weight of monomers of the formula III 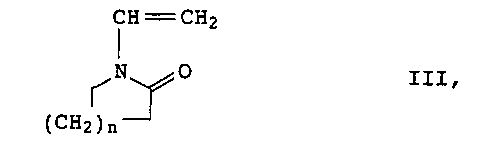 in which n is a number from 1 to 3, the weight ratio A:B being from 100:5 to 100:200, and at least partial neutralization of the graft copolymer.

2. A water-soluble or water-dispersible graft copolymer as claimed in claim 1, wherein the monomers
(A) consist, in an amount of
(a) from 65 to 85% by weight, of tert-butyl acrylate,
(b) from 15 to 25% by weight, of methacrylic acid and
(c) from 0 to 10% by weight, of stearyl methacrylate,
and are polymerized onto a prepolymer
(B) of the formula IV
―(Vcap)―(VP)―(M1)―(M2)― IV
where Vcap is vinylcaprolactam, VP is vinylpyrrolidone, M1 is an ester of methacrylic acid or acrylic acid with a C₁- to C₃₀-alcohol, or a C₁-C₃₀-alkylamide of methacrylic acid or acrylic acid, and M2 is a tert-amine-containing monomer, where the content
of Vcap is from 0 to 100% by weight,
of VP is from 0 to 100% by weiqht,
of M1 is from 0 to 50% by weight, and
of M2 is from 0 to 20% by weight,
with the proviso that the sum of Vcap and VP is at least 30% by weight, and the mass ratio (A) to (B) is from 100:20 to 100:70.

3. A water-soluble or water-dispersible graft copolymer as claimed in claim 1, wherein the prepolymer (B) is polyvinylcaprolactam.

4. A water-soluble or water-dispersible graft copolymer as claimed in claim 1, wherein the prepolymer (B) is polyvinylpyrrolidone.

5. A water-soluble or water-dispersible graft copolymer as claimed in claim 1, wherein the prepolymer (B) is a copolymer of vinylpyrrolidone and vinylcaprolactam.

6. A water-soluble or water-dispersible graft copolymer as claimed in claim 1, wherein the prepolymer (B) is a copolymer of vinylpyrrolidone and/or vinylcaprolactam and a monomer which contains a tertiary amine group.

7. A water-soluble or water-dispersible graft copolymer as claimed in claim 6, wherein the monomer which contains a tertiary amine group is a monomer of the formula V 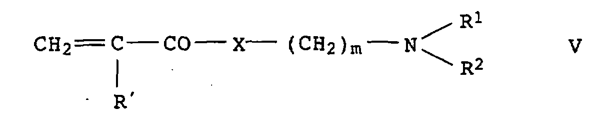 in which R' is hydrogen or a C₁- to C₆-alkyl radical, X is oxygen or an imino group, m is a number from 2 to 8, and R¹ and R² independently of one another are an alkyl radical having from 1 to 6 carbon atoms.

8. A process for the preparation of graft copolymers as claimed in claim 1, which comprises free-radically polymerizing the monomers
(A), consisting of
(a) from 50 to 85% by weight of monomers of the formula I 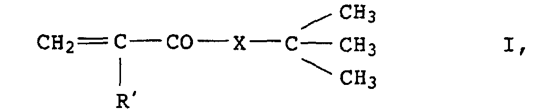 in which R' is hydrogen or a C₁- to C₆-alkyl radical, and X is the radical 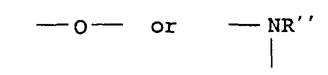 where R'' is hydrogen or a C₁- to C₆-alkyl radical, and
(b) from 15 to 30% by weight of a vinyl monomer which ha at least one carboxyl group, and, where appropriate,
(c) from 0 to 25% by weight of a free-radically polymerizable vinyl monomer of the formula II 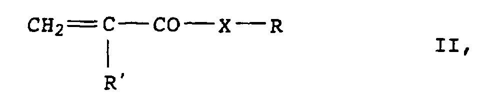 in which R is a C₆- to C₃₀-alkyl radical, and R' and X are as defined above, with
(B) a prepolymer with a K value of from 30 to 50, comprising, in copolymerized form, at least 30% by weight of monomers of the formula III 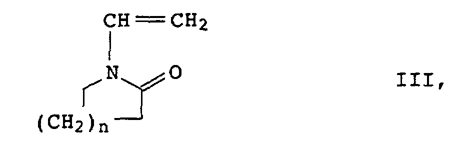 in which n is a number from 1 to 3,
the weight ratio A:B being from 100:5 to 100:200, and neutralizing at least some of the carboxyl groups of the graft copolymer.

9. The use of the water-soluble graft copolymer as claimed in any of claims 1 to 7 as a constituent of cosmetic preparations.

10. A hair cosmetic preparation comprising from 0.5 to 20% by weight of a graft copolymer as claimed in any of claims 1 to 7.

11. The use of the water-soluble graft copolymer as claimed in any of claims 1 and 7 as a coating or binder in pharmaceutical preparations.

12. A hair-setting preparation comprising
from 0.5 to 20% by weight of a graft copolymer as claimed in any of claims 1 to 7,
from 0 to 10% by weight of a standard commercial hair polymer,
from 50 to 99.5% by weight of an alcohol and/or water as solvent,
from 0 to 50% by weight of a propellant in the form of propane/ butane and/or dimethyl ether,
from 0 to 0.5% by weight of a water-soluble or dispersible silicone compound and
from 0 to 0.2% by weight of customary additives.

## Revendications

1. Copolymères greffés hydrosolubles ou hydrodispersables ayant une valeur K de 30 à 70, pouvant être obtenus par polymérisation radicalaire de monomères
(A) consistant pour l'essentiel en
(a) 50 à 85 % en poids de monomères de formule I 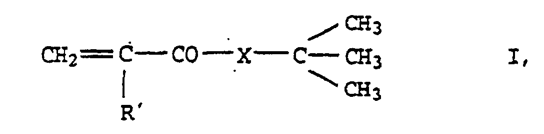 dans laquelle R' désigne l'hydrogène ou un reste alkyle en C₁ à C₅ et X le reste 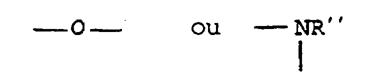 tandis que R" représente l'hydrogène ou un reste alkyle en C₁ à C₆, et
(b) 15 à 30 % en poids d'un monomère vinylique possédant au moins un groupe carboxyle, ainsi qu'éventuellement
(c) 0 à 25 % en poids d'un monomère vinylique polymérisable par voie radicalaire de formule II 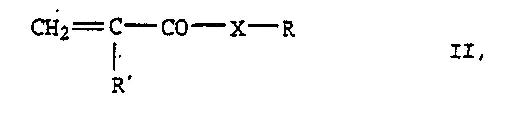
dans laquelle R désigne un reste alkyle en C₆ à C₃₀ et R' et X ont les significations susdites, avec
(B) un prépolymére ayant une valeur K de 30 à 50, contenant sous forme copolymérisée au moins 30 % en poids de monomères de formule III 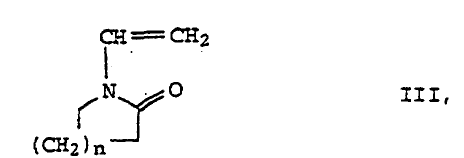
dans laquelle n représente le nombre 1 à 3, tandis que le rapport en poids A:B vaut 100:5 à 100:200, et au moins neutralisation partielle du copolymère greffé.

2. Copolymères greffés hydrosolubles ou hydrodispersables selon la revendication 1, **caractérisés par le fait que** les monomères
(A) consistent
(a) pour 65 à 85 % en poids en acrylate de tertbutyle,
(b) pour 15 à 25 % en poids en acide méthacrylique et
(c) pour 0 à 10 % en poids en méthacrylate de stéaryle
et sont copolymérisés sur un prépolymère
(B) de formule IV
―(Vcap)―(VP)―(M1)―(M2)― IV
tandis que Vcap désigne le caprolactame de vinyle, VP la pyrrolidone de vinyle, M1 un ester de l'acide méthacrylique ou de l'acide acrylique avec un alcool en C₁ à C₃₀, ou un amide d'alkyle en C₁-C₃₀ de l'acide méthacrylique ou de l'acide acrylique et M2 un monomère contenant une amine tertiaire, tandis que la proportion
de Vcap est de 0 à 100 % en poids,
de VP est de 0 à 100 % en poids,
de M1 est de 0 à 50 % en poids, et
de M2 est de 0 à 20 % en poids,
avec pour condition que la somme de Vcap et VP vaut au moins 30 % en poids et le rapport en masse de (A) à (B) vaut 100:20 à 100:70.

3. Copolymères greffés hydrosolubles ou hydrodispersables selon la revendication 1, **caractérisés par le fait que** le prépolymère (B) est du poly(caprolactame de vinyle).

4. Copolymères greffés hydrosolubles ou hydrodispersables selon la revendication 1, **caractérisés par le fait que** le prépolymère (B) est de la poly(pyrrolidone de vinyle).

5. Copolymères greffés hydrosolubles ou hydrodispersables selon la revendication 1, **caractérisés par le fait que** prépolymère (B) est un copolymère de pyrrolidone de vinyle et de caprolactame de vinyle.

6. Copolymères greffés hydrosolubles ou hydrodispersables selon la revendication 1, **caractérisés par le fait que** prépolymère (B) est un copolymère de pyrrolidone de vinyle et/ou de caprolactame de vinyle et d'un monomère contenant un groupe amine tertiaire.

7. Copolymères greffés hydrosolubles ou hydrodispersables selon la revendication 6, **caractérisés par le fait que** le monomère contenant un groupe amine tertiaire est un monomère de formule V 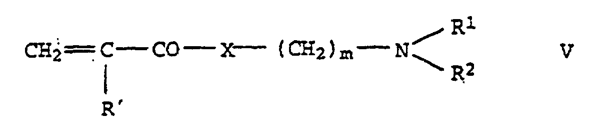 dans laquelle R' désigne l'hydrogène ou un reste alkyle en C₁ à C₆, X représente l'oxygène ou un groupe imino, m désigne les nombres 2 à 8 et R¹ et R² sont indépendamment l'un de l'autre un reste alkyle ayant 1 à 6 atomes de carbone.

8. Procédé pour la préparation de copolymères greffés selon la revendication 1, **caractérisé par le fait qu'**on polymérise par voie radicalaire les monomères
(A) consistant en
(a) 50 à 85 % en poids de monomères de formule I 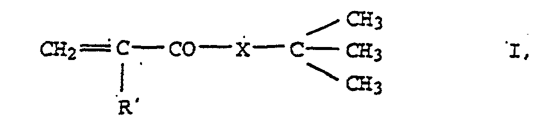 dans laquelle R' désigne l'hydrogène ou un reste alkyle en C₁ à C₅ et X le reste 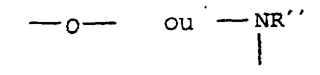 tandis que R" représente l'hydrogène ou un reste alkyle en C₁ à C₆, et
(b) 15 à 30 % en poids d'un monomère vinylique possédant au moins un groupe carboxyle, ainsi gu'éventuellement
(c) 0 à 25 % en poids d'un monomère vinylique polymérisable par voie radicalaire de formule II 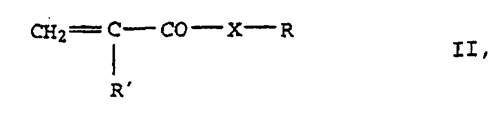
dans laquelle R désigne un reste alkyle en C₆ à C₃₀ et R' et X ont les significations susdites, avec
(B) un prépolymère ayant une valeur K de 30 à 50, contenant sous forme copolymérisée au moins 30 % en poids de monomères de formule III 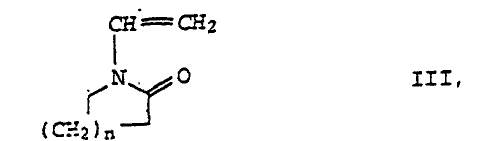
dans laquelle n représente le nombre 1 à 3, tandis que le rapport en poids A:B vaut 100:5 à 100:200, et on neutralise au moins une partie des groupes carboxyle du copolymère greffé.

9. Utilisation des copolymères greffés selon l'une des revendications 1 à 7 comme composant de préparations cosmétiques.

10. Préparation cosmétique capillaire, contenant 0,5 à 20 % en poids d'un copolymère greffé selon les revendications 1 à 7.

11. Utilisation de copolymères greffés hydrosolubles selon l'une des revendications 1 à 7 comme agents d'enduction ou de liaison dans des préparations pharmaceutiques.

12. Préparation de fixateur pour cheveux, contenant
0,5 à 20 % en poids d'un copolymère greffé selon l'une des revendications 1 à 7,
0 à 10 % en poids d'un polymère capillaire classique du commerce,
50 à 99,5 % en poids d'un alcool et/ou d'eau comme solvant,
0 à 50 % en poids d'un agent propulseur sous forme de propane/butane et/ou d'éther diméthylique,
0 à 0,5 % en poids d'un composé de silicone hydrosoluble ou hydrodispersable, et
0 à 0,2 % en poids d'additifs classiques.
